# EUROPÄISCHE PATENTANMELDUNG

(11) **EP 4 813 336 A1**
(43) Veröffentlichungstag der Anmeldung: **30.09.2026**
(21) Anmeldenummer: 25165673.2
(22) Anmeldetag: 24.03.2025
(51) Int. Cl.: A61B 34/20, A61B 90/20, A61B 90/00

(54) **NAVIGATIONSSYSTEM, MEDIZINISCHES SYSTEM, NAVIGATIONSVERFAHREN UND VERWENDUNG**

(71) Anmelder: B. Braun New Ventures GmbH, 79110 Freiburg im Breisgau (DE)
(72) Erfinder: STEHLE, Simon, 79100 Freiburg (DE); STAWIASKI, Jean, 79117 Freiburg (DE)
(74) Vertreter: Winter, Brandl - Partnerschaft mbB

(57) **Zusammenfassung**

Die Offenbarung betrifft ein Navigationssystem (2) zur Nachverfolgung eines medizinischen Instruments (4) relativ zu einem Patienten (P), mit einer ersten Navigationskamera (6), die eingerichtet ist, eine aktuelle Position und/oder Orientierung des medizinischen Instruments (4) im sichtbaren Spektrum zu erfassen, einer zweiten Navigationskamera (8), die eingerichtet ist, eine aktuelle Position und/oder Orientierung des Patienten (P) im sichtbaren Spektrum zu erfassen, und einer Steuereinheit (10), die eingerichtet ist, aus der von der ersten Navigationskamera (6) erfassten aktuellen Position und/oder Orientierung des medizinischen Instruments (4) und der von der zweiten Navigationskamera (8) erfassten aktuellen Position und/oder Orientierung des Patienten (P) eine aktuelle Relativposition und/oder -Orientierung des medizinischen Instruments (4) zu dem Patienten (P) zu bestimmen. Ferner betrifft die Offenbarung ein medizinisches System (16), ein Navigationsverfahren (30) und eine Verwendung.

## Beschreibung

### Technisches Gebiet

Die vorliegende Offenbarung betrifft ein Navigationssystem zum Nachverfolgen eines medizinischen Instruments relativ zu einem Patienten. Navigationssysteme sind in der computergestützten bzw. computerassistierten Chirurgie weit verbreitet und helfen einem Chirurgen dabei, sich während einer Operation zu orientieren.

### Hintergrund der Offenbarung

Navigationssysteme werden verwendet, um Objekte während einer Operation nachzuverfolgen (tracking), also um eine (möglichst) zeitaktuelle Position und/oder Orientierung zu erfassen. Beispielsweise können medizinische Instrumente zu einem Patienten nachverfolgt werden, um die derzeitige bzw. aktuelle Position und/oder Orientierung des Instruments zu einem Bereich von Interesse zu bestimmen. In der Neurochirurgie kann z.B. eine Biopsie-Nadel relativ zu einem Patienten nachverfolgt werden, um sie präzise zu einem Hirn-Tumor des Patienten zu navigieren.

Navigationssysteme weisen dazu einen Sensor auf, der die Position und/oder Orientierung eines Objekts, z.B. des Patienten oder eines Instruments, erfassen kann. Der Sensor kann z.B. eine Kamera sein, dann spricht man von optischem Tracking. Typischerweise sind Navigationskameras für das infrarote (IR) Spektrum ausgelegt. Das nachzuverfolgende Objekt weist einen Tracker auf, in diesem Fall mit IR-Markern, die seine Position und/oder Orientierung referenzieren und von der IR-Navigationskamera erfasst werden können. Die IR-Marker sind typischerweise kugelförmig und können aktiv oder passiv ausgestaltet sein, d.h. sie können selbst IR-Licht abstrahlen oder IR-Licht der Navigationskamera reflektieren.

Alternativ kann die Navigationskamera auch zur Nachverfolgung im sichtbaren Spektrum (für Menschen sichtbare Wellenlängen) ausgelegt sein. Das nachzuverfolgende Objekt kann dann z.B. anhand seiner Form nachverfolgt werden, oder ein optisches Muster, wie z.B. einen Barcode, aufweisen, der von der Navigationskamera erfasst und nachverfolgt werden kann, um damit das Instrument nachzuverfolgen.

Optische Navigationskameras haben aber häufig Sichtprobleme. Um ein Instrument relativ zu einem Patienten nachverfolgen zu können, sollte die Navigationskamera aber möglichst immer sowohl das Instrument als auch den Patienten sehen können. Normalerweise wird der Patient über einen Patienten-Tracker nachverfolgt, der rigide an dem Patienten angebracht wird und seine Position und/oder Orientierung bzw. eines anatomischen Bereichs von Interesse, z.B. eines Wirbels oder des Schädels etc., referenzieren kann. Der Patienten-Tracker kann z.B. ebenfalls IR-Marker aufweisen, die von der IR-Navigationskamera erfasst werden können. Es versteht sich, dass die Navigationskamera also nicht unbedingt den Patienten selbst, aber zumindest den Patienten-Tracker und das Instrument sehen sollte.

Insbesondere bei Eingriffen an der Wirbelsäule oder dem Kopf kann die Sicht der Navigationskamera auf einen Patienten-Tracker und ein medizinisches Instrument aber z.B. durch einen Chirurgen oder andere Instrumente eingeschränkt sein.

Typischerweise werden Navigationskameras deshalb in einiger Entfernung zum Operationsbereich und meist oberhalb des Patienten positioniert, um einen guten Überblick zu haben. Je weiter aber die Navigationskamera entfernt ist, desto größer müssen die Tracker sein, um sie noch gut erkennen zu können. Größere Tracker sind jedoch unhandlicher und können ggfls. den Chirurgen beeinträchtigen.

Insbesondere in der Wirbelsäulenchirurgie kann es erforderlich sein, mehrere Wirbel einzeln nachverfolgen zu können, sodass an jedem Wirbel jeweils ein Patienten-Tracker angebracht wird, was insbesondere im Fall von großen und unhandlichen Trackern aufgrund des begrenzten Platzes zu Problemen führen kann und somit nachteilhaft ist. Zudem steigt mit der Anzahl der Patienten-Trackern die Wahrscheinlichkeit, dass bei einem der Patienten-Tracker die Sichtverbindung zur Navigationskamera blockiert wird.

Darüber hinaus steigt mit der Größe des Patienten-Trackers das Risiko, dass medizinisches Personal während der Operation gegen den Patienten-Tracker stoßen kann. Dadurch kann der Patient verletzt oder eine Pose des Patienten-Tracker zum Patienten verändert werden, wodurch wiederum eine vorherige Registrierung ungültig wird und der Patienten-Tracker nicht mehr zur Navigation verwendet werden kann.

### Kurzbeschreibung der Offenbarung

In Anbetracht der oben beschriebenen Problematik ist es daher eine Aufgabe der Offenbarung, die Nachteile aus dem Stand der Technik zu vermeiden oder zumindest zu verringern. Insbesondere soll eine Möglichkeit zur Nachverfolgung von einem medizinischen Instrument zu einem Patienten bereitgestellt werden, bei der möglichst keine oder geringere Sichtprobleme auftreten und die möglichst wenig fehleranfällig, etwa durch unbeabsichtigtes Anstoßen des Patienten-Trackers, ist.

Diese Aufgabe wird durch ein Navigationssystem mit den Merkmalen des Anspruchs 1, sowie ein medizinisches System, ein Verfahren und eine Verwendung gemäß den nebengeordneten Ansprüchen gelöst. Vorteilhafte Ausführungsformen sind Gegenstand der abhängigen Ansprüche und/oder nachfolgend in der Beschreibung beschrieben.

Das offenbarungsgemäße Navigationssystem dient zur Nachverfolgung eines medizinischen Instruments relativ zu einem Patienten. Das Navigationssystem weist eine erste Navigationskamera auf, die eingerichtet ist, eine aktuelle Position und/oder Orientierung des medizinischen Instruments im sichtbaren Spektrum zu erfassen. Das Navigationssystem weist eine (zu der ersten Navigationskamera separate) zweite Navigationskamera auf, die eingerichtet ist, eine aktuelle Position und/oder Orientierung des Patienten im sichtbaren Spektrum zu erfassen. Das Navigationssystem weist eine Steuereinheit auf, die eingerichtet ist, aus der von der ersten Navigationskamera erfassten aktuellen Position und/oder Orientierung des medizinischen Instruments und der von der zweiten Navigationskamera erfassten aktuellen Position und/oder Orientierung des Patienten eine aktuelle Relativposition und/oder -Orientierung des medizinischen Instruments zu dem Patienten zu bestimmen.

Es ist also ein Kerngedanke der Offenbarung zwei separate Kameras zur Nachverfolgung eines medizinischen Instruments zu einem Patienten zu verwenden, wobei die eine Kamera das medizinische Instrument nachverfolgt und die andere Kamera den Patienten nachverfolgt. Somit ist es nicht notwendig, dass eine Kamera den Patienten und das Instrument gleichzeitig sieht, um die Relativposition und/oder - Orientierung des Instruments zum Patienten zu bestimmen. Das heißt, dass die Kameras vorzugsweise so angeordnet sind, dass sich der Patient außerhalb eines Sichtfelds/einer direkten Sichtlinie der das medizinische Instrument nachverfolgenden Kamera befindet oder sich das medizinische Instrument außerhalb eines Sichtfelds/ einer direkten Sichtlinie der den Patienten nachverfolgenden Kamera befindet. Die Steuereinheit kann z.B. dazu eingerichtet sein, basierend auf der (bestimmten) Relativposition und/oder -Orientierung das medizinische Instrument positions- und/oder orientierungsgetreu auf einer, z.B. präoperativen, radiologischen Aufnahme des Patienten darzustellen. Mit anderen Worten ist die Steuereinheit eingerichtet, die aktuelle Relativposition und/oder -Orientierung zwischen dem medizinischen Instrument und dem Patienten auf Basis der das medizinische Instrument enthaltenden Aufnahme der ersten Navigationskamera, der den Patienten enthaltenden Aufnahme der zweiten Navigationskamera und einer bekannten Transformation (d.h. Relativposition und/oder -Orientierung) zwischen den zwei Navigationskameras zu bestimmen.

Es versteht sich, dass die Navigationskameras den Patienten bzw. das Instrument nachverfolgen können, wenn sich diese oder z.B. ein damit verbundener Tracker im Sichtfeld der jeweiligen Kamera befindet.

Es versteht sich, dass die Position und/oder Orientierung des Patienten auch indirekt über einen Patienten-Tracker erfasst werden kann, der Patient selbst kann dann also auch außerhalb des Sichtfelds der zweiten Navigationskamera sein. In anderen Worten ist bevorzugt die zweite Navigationskamera dazu eingerichtet, einen Patienten-Tracker, vorzugsweise auch mehrere gleichzeitig und individuell, nachzuverfolgen.

Die erste Navigationskamera kann eingerichtet sein, das medizinische Instrument selbst, z.B. über die Form, nachzuverfolgen. Bevorzugt ist die erste Navigationskamera aber eingerichtet, das medizinische Instrument über ein optisches Muster des medizinischen Instruments nachzuverfolgen.

Es ist zu verstehen, dass das optische Muster, und jedes optische Muster in dieser Offenbarung, zum Nachverfolgen im sichtbaren Spektrum (für Menschen sichtbares Spektrum) angepasst ist. Dazu kann es unterschiedliche Abschnitte bzw. Merkmale (sogenannte Features) in zumindest zwei unterschiedlichen Farbtönen, z.B. schwarz und weiß, aufweisen bzw. Kontrast aufweisen. Es handelt sich also explizit nicht um IR-Marker. Das optische Muster kann beispielsweise Striche (wie ein Strichcode), Quadrate (wie ein QR-Code) und/oder andere Formen wie Kreise oder Ellipsen aufweisen, welche insbesondere regelmäßig oder auch unregelmäßig angeordnet sein können.

Es versteht sich, dass die erste Navigationskamera eingerichtet sein kann, mehrere medizinische Instrumente insbesondere gleichzeitig und individuell nachzuverfolgen.

Die erste Navigationskamera und die zweite Navigationskamera sind für das sichtbare Spektrum ausgelegt. Es versteht sich, dass sie dadurch bevorzugt eingerichtet sind, optische Aufnahmen, also Bilder und/oder Bildsequenzen bzw. Videos (im sichtbaren Spektrum) zu erstellen. Bevorzugt weist das Navigationssystem entsprechend eine Anzeigevorrichtung auf, die eingerichtet ist, Aufnahmen der ersten und zweiten Navigationskamera anzuzeigen. Die Anzeigevorrichtung kann z.B. ein Monitor oder ein Head-Display sein.

Das medizinische Instrument ist bevorzugt ein chirurgisches Instrument, also für einen chirurgischen Eingriff ausgelegt. Es kann sich dabei z.B. ein Messinstrument, wie eine Elektrode zur Stereoelektroenzephalographie (sEEG), ein bildgebendes Instrument wie ein Endoskop, oder einen Manipulator zum Manipulieren des Patienten wie z.B. einen (Knochen-) Bohrer oder Fräser handeln. Auch ein Implantat kann unter dem medizinischen Instrument miteingeschlossen sein.

Das Navigationssystem ist bevorzugt dazu ausgelegt bzw. eingerichtet, das medizinische Instrument zum Patienten räumlich in sechs Freiheitsgraden (in drei translatorischen und drei rotarischen Freiheitsgraden) nachzuverfolgen (6D-Tracking). Die Offenbarung ist aber nicht darauf beschränkt, es kann zum Beispiel auch nur die Relativposition (in drei kartesischen Koordinaten) des Instruments zum Patienten erfasst bzw. bestimmt werden.

Die Vorteile der Offenbarung bestehen darin, dass zwei einzelne Navigationskameras (im Folgenden auch nur als Kameras bezeichnet) verwendet werden, um jeweils einzeln ein Instrument und einen Patienten nachzuverfolgen, um damit das Instrument zum Patienten nachzuverfolgen. Damit können die zwei Kameras flexibler als eine einzige Kamera, die Instrument und Patient gleichzeitig sehen muss, angeordnet werden, sodass Sichtprobleme besser vermieden werden. Dadurch wird auch ermöglicht, einen Abstand zwischen dem Instrument und dem Patienten-Tracker zu vergrößern und gleichzeitig die Nachverfolgung des Instruments zum Patienten sicherzustellen. Wie oben beschrieben sind beide Kameras für das sichtbare Spektrum ausgelegt. Dies hat zum einen den Vorteil, dass Aufnahmen der Kameras zur Bildunterstützung während des Eingriffs verwendet werden können. Zum anderen sind Tracker mit IR-Markern normalerweise größer und unhandlicher, wohingegen optische Muster zur Nachverfolgung im sichtbaren Spektrum kleiner sein können und z.B. auf das Instrument aufgeklebt oder eingraviert werden können, und somit die effektive Größe des Instruments nicht/ kaum verändern. Ein weiterer Vorteil ist, dass die zwei Kameras unterschiedliche Optiken aufweisen bzw. unterschiedlicher Art sein können, und damit auf ihre jeweilige Aufgabe individuell angepasst sein können. Die erste Navigationskamera kann also gezielt dafür ausgewählt bzw. angepasst sein, besonders gut ein medizinisches Instrument nachverfolgen zu können, wohingegen die zweite Navigationskamera also gezielt dafür ausgewählt bzw. angepasst sein kann, besonders gut den Patienten nachzuverfolgen.

Bevorzugt sind die erste Navigationskamera und die zweite Navigationskamera fest zueinander angeordnet bzw. aneinander befestigt. Somit können die erfassten Positionsdaten des Patienten und des medizinischen Instruments leicht ausgetauscht bzw. in ein gemeinsames Koordinatensystem umgerechnet werden, da die Transformation zwischen den beiden Kameras damit bekannt ist. In anderen Worten sind die erste Navigationskamera und die zweite Navigationskamera bevorzugt zueinander unbeweglich angeordnet.

Bevorzugt sind die erste Navigationskamera und die zweite Navigationskamera, insbesondere als ein Endeffektor, gemeinsam an einem Roboterarm angebracht. Somit können sie beide gemeinsam bewegt bzw. positioniert werden. Bevorzugt sind die erste Navigationskamera und die zweite Navigationskamera beide extrakorporal.

Bevorzugt sind die erste Navigationskamera und die zweite Navigationskamera in dieselbe Richtung ausgerichtet, blicken also sozusagen in dieselbe Richtung (möglichst) parallel zueinander. Eine optische Achse der ersten Navigationskamera und eine optische Achse der zweiten Navigationskamera sind also, zumindest im Wesentlichen, parallel zueinander.

Bevorzugt überlappen sich ein Sichtfeld der ersten Navigationskamera und ein Sichtfeld der zweiten Navigationskamera.

Es versteht sich, dass die erste Navigationskamera und die zweite Navigationskamera bevorzugt jeweils und/oder zueinander kalibriert oder kalibrierbar sind.

Bevorzugt unterscheiden sich die erste Navigationskamera und die zweite Navigationskamera in ihrer Art voneinander. Bevorzugt weist die erste Navigationskamera eine Optik bzw. optische Komponenten auf, die sich von der Optik bzw. den optischen Komponenten der zweiten Navigationskamera unterscheiden.

Bevorzugt ist die erste Navigationskamera ein digitales Operationsmikroskop. Die erste Navigationskamera ist demnach bevorzugt ein digitales Mikroskop (also ein Mikroskop, das eingerichtet ist, eine digitale Bildaufnahme zu erstellen, welche auf einem Monitor ausgegeben werden kann), das für die Chirurgie bzw. zum Operieren angepasst ist. Das Mikroskop kann auch hybrides Mikroskop sein, das zusätzlich eine Optik zum direkten Betrachten eines darunter befindlichen Objekts aufweist. Das hat den Vorteil, dass die erste Navigationskamera verwendet werden kann, um einen Operationsbereich hochauflösend und vergrößert darzustellen und gleichzeitig dabei das medizinische Instrument nachzuverfolgen, welches bzw. wenn es am Operationsbereich verwendet wird.

Bevorzugt ist die erste Navigationskamera hochauflösend, vorzugsweise mit einem kleinen Sichtfeld, insbesondere im Vergleich zu der zweiten Navigationskamera. Vorzugsweise weist die erste Navigationskamera einen variablen Fokus auf. Damit kann sie auf das Instrument und/oder einen Operationsbereich präzise scharf gestellt werden. Damit kann das Instrument ein optisches Muster mit sehr feinen und/oder kleinen Merkmalen aufweisen, das von der hochauflösenden ersten Navigationskamera immer noch gut erkannt werden kann.

Bevorzugt ist die zweite Navigationskamera eine weitwinklige Kamera, insbesondere im Vergleich zu der ersten Navigationskamera. Bevorzugt hat die zweite Navigationskamera ein größeres Sichtfeld als die erste Navigationskamera. Damit kann die zweite Navigationskamera einen größeren (Bild-) Bereich aufnehmen und eine Übersichtsaufnahme liefern, die einen größeren Bereich als die erste Navigationskamera zeigt. Außerdem kann sie damit ggfls. mehrere Patienten-Tracker gleichzeitig erfassen und nachverfolgen, die zueinander beanstandet am Patienten, z.B. an einzelnen Wirbeln, angebracht sind.

Bevorzugt weist die zweite Navigationskamera einen festen (bzw. unveränderbaren) Fokus bzw. Fokustiefe auf. Bevorzugt weist die zweite Navigationskamera (dazu) eine große Tiefenschärfe, insbesondere im Vergleich zur ersten Navigationskamera, auf. Das hat den Vorteil, dass die zweite Navigationskamera ohne aufwendig den Fokus einzustellen (möglichst immer) ausreichend scharf bzw. präzise eingestellt sein kann.

Bevorzugt liegt ein/ das Sichtfeld der ersten Navigationskamera in einem/ dem Sichtfeld der zweiten Navigationskamera (zumindest ab einem bestimmten Abstand bzw. Tiefe von der zweiten Navigationskamera). In anderen Worten umschließt bevorzugt das größere Sichtfeld der zweiten Navigationskamera das darin liegende kleinere Sichtfeld der ersten Navigationskamera (aber einer gewissen Tiefe). Damit kann die erste Navigationskamera dann z.B. eine Aufnahme eines kleinen Bereichs von Interesse, z.B. eines Operationsbereiches, erfassen und die zweite Navigationskamera eine Aufnahme auch der umliegenden Strukturen zur Übersicht liefern.

Bevorzugt ist die erste Navigationskamera stereoskopisch. Bevorzugt ist zweite Navigationskamera stereoskopisch.

Ferner betrifft die Offenbarung ein medizinisches System mit einem Navigationssystem, vorzugsweise wie voranstehend beschrieben, und einem Patienten-Tracker. Der Patienten-Tracker ist dazu eingerichtet, an einem/ dem Patienten rigide befestigt zu werden, um dessen (aktuelle) Position und/oder Orientierung zu referenzieren. Der Patienten-Tracker weist ein optisches Muster im sichtbaren Spektrum auf. Der Patienten-Tracker ist damit also zur Nachverfolgung im sichtbaren Spektrum angepasst. Die zweite Navigationskamera ist eingerichtet, das optische Muster des Patienten-Trackers visuell zu erfassen. Die zweite Navigationskamera ist eingerichtet, den Patienten über den Patienten-Tracker bzw. das optische Muster des Patienten-Trackers (im sichtbaren Spektrum) nachzuverfolgen bzw. eine aktuelle Position und/oder Orientierung des Patienten über den Patienten-Tracker zu erfassen.

Es versteht sich, dass der Patienten-Tracker auch indirekt mit dem Patienten rigide verbunden bzw. befestigt werden kann, beispielsweise über eine Kopfklemme. Der Patienten-Tracker kann also eingerichtet sein, direkt an den Patienten rigide befestigt zu werden, und/oder eingerichtet sein, z.B. an eine Patientenbefestigungsvorrichtung, wie eine Kopfklemme, die eingerichtet ist, selbst rigide an dem Patienten angebracht zu werden, rigide befestigt zu werden. Der Patienten-Tracker weist bevorzugt einen Befestigungsabschnitt zum rigiden Befestigen des Patienten-Trackers am Patienten auf. Der Befestigungsabschnitt kann z.B. eine Schraube oder eine Klebefläche sein. Rigide bedeutet, dass (möglichst) keine Relativbewegung zwischen dem Patienten und dem Patienten-Tracker auftritt, damit die Position und/oder Orientierung des Patienten korrekt, insbesondere nach einer initialen Registrierung, referenziert wird.

Es versteht sich, dass das medizinische System mehrere (solcher) Patienten-Tracker aufweisen kann und/oder die zweite Navigationskamera eingerichtet sein kann, mehrere Patienten-Tracker, insbesondere gleichzeitig und individuell, nachzuverfolgen, insbesondere um mehrere anatomische Strukturen bzw. Bereiche des Patienten gleichzeitig und individuell bzw. einzeln nachzuverfolgen.

Bevorzugt weist das medizinische System ein medizinisches Instrument, vorzugsweise wie voranstehend bereits beschrieben, auf, wobei das medizinische Instrument ein optisches Muster im sichtbaren Spektrum aufweist und die zweite Navigationskamera eingerichtet ist, das optische Muster des medizinischen Instruments visuell zu erfassen. Bevorzugt ist die zweite Navigationskamera eingerichtet, das medizinischen Instrument über sein optisches Muster (im sichtbaren Spektrum) nachzuverfolgen bzw. darüber eine aktuelle Position und/oder Orientierung des medizinischen Instruments zu bestimmen.

Bevorzugt ist das optische Muster des medizinischen Instruments eingraviert z.B. mittels eines Lasers, und/oder aufgeklebt. Damit vergrößert es das medizinische Instrument nicht bzw. nur sehr gering.

Alternativ könnte das medizinische Instrument auch kein optisches Muster aufweisen und/oder die zweite Navigationskamera eingerichtet sein, das medizinische Instrument z.B. anhand seiner Form nachzuverfolgen.

Bevorzugt unterscheiden sich das optische Muster des Instruments und das optische Muster des Patienten-Trackers in ihrer Beschaffenheit voneinander.

Beispielsweise können sie sich in ihrer Größe, in der Farbe und/oder Art der Merkmale (z.B. Striche oder Kreise) oder auch darin unterscheiden, wie viele Freiheitsgrade des Instruments bzw. des Patienten-Trackers sie angeben können. Beispielsweise kann das optische Muster des Patienten-Trackers für eine 5D-Nachverfolgung (5D-Tracking) ausgelegt sein, da es zum Beispiel rotationssymmetrisch ist, und dagegen das optische Muster des Instruments für eine 6D-Nachverfolgung (6D-Tracking) ausgelegt sein.

Bevorzugt ist das optische Muster des medizinischen Instruments wiederverwendbar und dazu sterilisierbar. Dazu kann es z.B. eingraviert z.B. gelasert sein und/oder beschichtet sein. Dagegen kann der Patienten-Tracker ein Einwegprodukt sein und/oder das optische Muster des Patienten-Trackers nicht sterilisierbar sein. Das optische Muster des Patienten-Trackers kann z.B. aufgeklebt oder eingraviert sein.

Bevorzugt ist das optische Muster des medizinischen Instruments gelasert bzw. eingraviert, insbesondere mit höherer Energie bzw. tiefer als das optische Muster des Patienten-Trackers, um eine geringere Abrasivität zu haben.

Bevorzugt ist das optische Muster des medizinischen Instruments im Vergleich zu dem optischen Muster des Patienten-Tracker weniger reflektierend bzw. weist einen geringeren Reflexionsgrad auf. Dies kann z.B. durch dunklere Farbe oder eine Beschichtung erreicht werden. Damit kann es geeigneter sein, unter einem Operationsmikroskop verwendet zu werden.

Bevorzugt ist das optische Muster des medizinischen Instruments im Vergleich zu dem optischen Muster des Patienten-Tracker feiner und/oder kleiner.

Das optische Muster des medizinischen Instruments ist vorzugsweise also im Vergleich zu dem optischen Muster des Patienten-Tracker in seiner Größe, insbesondere ein Abschnitt der aus einer Richtung sichtbar ist, geringer. Das hat den Vorteil, dass es platzsparend sein kann und direkt am Instrument angebracht sein kann. Der Patienten-Tracker kann größer sein, da er meist nicht so nah oder direkt wie ggfls. das Instrument am Operationsbereich ist und daher die Ergonomie für den Arzt weniger beeinflusst.

Insbesondere da die erste Navigationskamera bevorzugt ein Operationsmikroskop (mit hoher Auflösung) ist, kann das optische Muster des Patienten-Tracker feiner sein bzw. feinere Strukturen bzw. Merkmale aufweisen, wodurch es auch insgesamt kleiner werden kann.

Bevorzugt ist das optische Muster des medizinischen Instruments, insbesondere im Vergleich zu dem optischen Muster des Patienten-Tracker, robust gegen eine (teilweise) Verdeckung. Das heißt, dass das optische Muster des medizinischen Instruments bevorzugt dazu angepasst ist, dass in dem Fall, wenn ein Teil des optischen Musters, z.B. durch Blut während einer Operation oder durch einen Chirurgen, verdeckt ist, es weiterhin möglichst genug freiliegende Merkmale (features) aufweist, die genügend Information zum Referenzieren der Position und/oder Orientierung des medizinischen Instruments bereitstellen, sodass die erste Navigationskamera (möglichst) das Instrument weiterhin nachverfolgen kann.

Bevorzugt weist das optische Muster des medizinischen Instruments dazu Redundanzen, insbesondere mehr als das optische Muster des Patienten-Trackers, auf.

Bevorzugt weist das medizinische System eine (zu der ersten Navigationskamera und der zweiten Navigationskamera separate) dritte Navigationskamera für das infrarote Spektrum und zumindest einen IR-Marker, der mit dem Roboterarm verbunden ist, auf. Die dritte Navigationskamera ist eingerichtet, den IR-Marker im infraroten Spektrum nachzuverfolgen, insbesondere um darüber eine Position und/oder Orientierung des Roboterarm, vorzugsweise der ersten und zweiten Navigationskamera, zu erfassen.

Der IR-Marker kann dabei z.B. am Roboterarm selbst, am Endeffektor, oder an einer Basis des Roboterarms angebracht sein. Somit kann eine Position und/oder Orientierung des Roboterarms, insbesondere der der ersten und zweiten Navigationskamera, ggfls. über eine Roboterkinematik, bestimmt werden, beispielsweise um eine andere medizinische Vorrichtung wie z.B. einen C-Bogen zur Bildgebung relativ dazu zu positionieren und/oder für eine Registrierung des Patienten.

Es versteht sich, dass die dritte (IR-) Navigationskamera beabstandet zum Roboterarm positioniert oder positionierbar ist.

Ferner betrifft die Offenbarung ein Navigationsverfahren zur Nachverfolgung eines medizinischen Instruments relativ zu einem Patienten, vorzugsweise für ein Navigationssystem und/oder ein medizinisches System wie voranstehend beschrieben, mit den Schritten:
- Erfassen einer aktuellen Position und/oder Orientierung des medizinischen Instruments im sichtbaren Spektrum, vorzugsweise über ein optisches Muster des medizinischen Instruments, durch eine/ die erste Navigationskamera;
- Erfassen einer aktuellen Position und/oder Orientierung des Patienten im sichtbaren Spektrum, vorzugsweise über einen Patienten-Tracker mit einem optischen Muster, durch eine/die zweite, separate Navigationskamera; und
- Bestimmen einer aktuellen Relativposition und/oder -Orientierung des medizinischen Instruments zu dem Patienten aus der von der ersten Navigationskamera erfassten aktuellen Position und/oder Orientierung des medizinischen Instruments und der von der zweiten Navigationskamera erfassten aktuellen Position und/oder Orientierung des Patienten (insbesondere durch eine/ die Steuereinheit).

Es versteht sich, dass insbesondere die ersten beiden genannten Schritte bevorzugt gleichzeitig ausgeführt werden und vorzugsweise der dritte Schritt auch möglichst schnell danach (möglichst gleichzeitig) ausgeführt wird. In anderen Worten wird das Verfahren bevorzugt (möglichst) echtzeitfähig ausgeführt.

Es sei angemerkt, dass das Verfahren also maschinell ist und z.B. durch ein entsprechendes Navigationssystem ausgeführt wird bzw. ausgeführt werden kann, und somit einen Arzt selbst nicht in seiner Tätigkeit einschränkt und keinen chirurgischen oder therapeutischen Schritt aufweist.

Gemäß einem ggfls. unabhängig zu beanspruchenden Aspekt betrifft die Offenbarung ferner ein Navigationssystem mit einer ersten Navigationskamera, einer zweiten Navigationskamera und vorzugsweise einer Steuereinheit, vorzugsweise wie voranstehend beschrieben, wobei das Navigationssystem eingerichtet ist, die Schritte des Verfahrens, wie voranstehend beschrieben, auszuführen.

Gemäß einem ggfls. unabhängig zu beanspruchenden Aspekt betrifft die Offenbarung ferner ein computerlesbares Speichermedium, das Funktionen aufweist, die ein Navigationssystem und/oder medizinisches System, vorzugsweise wie voranstehend beschrieben, veranlassen, die Schritte des Verfahrens, wie voranstehend beschrieben, auszuführen.

Ferner betrifft die Offenbarung eine Verwendung einer ersten Navigationskamera zum Erfassen einer aktuellen Position und/oder Orientierung eines medizinischen Instruments im sichtbaren Spektrum und einer separaten zweiten Navigationskamera zum Erfassen einer aktuellen Position und/oder Orientierung eines Patienten im sichtbaren Spektrum, um das medizinische Instrument relativ zu dem Patienten nachzuverfolgen, oder in anderen Worten zum Bestimmen einer aktuellen Relativposition und/oder -Orientierung des medizinischen Instruments zu dem Patienten.

### Kurzbeschreibung der Figuren

Die Offenbarung wird im Folgenden anhand bevorzugter Ausführungsformen und unter Bezugnahme auf die beigefügten Figuren näher erläutert.
Fig. 1 zeigt ein Navigationssystem sowie ein medizinisches System gemäß der vorliegenden Offenbarung in einer bevorzugten Ausführungsform beispielhaft bei einem spinalen Eingriff;
Fig. 2 zeigt das Navigationssystem und ein medizinisches System aus Fig. 1 bei einem neuronalen Eingriff an einem liegenden Patienten;
Fig. 3 zeigt ein Navigationssystem und ein medizinisches System gemäß der vorliegenden Offenbarung bei einem Eingriff an einem halb-sitzenden Patienten;
Fig. 4 zeigt ein Navigationsverfahren gemäß der vorliegenden Offenbarung.

Die Figuren sind schematisch und dienen lediglich dem Verständnis der Offenbarung. Die Merkmale der verschiedenen Ausführungsformen können untereinander ausgetauscht werden.

### Detaillierte Beschreibung der bevorzugten Ausführungsformen

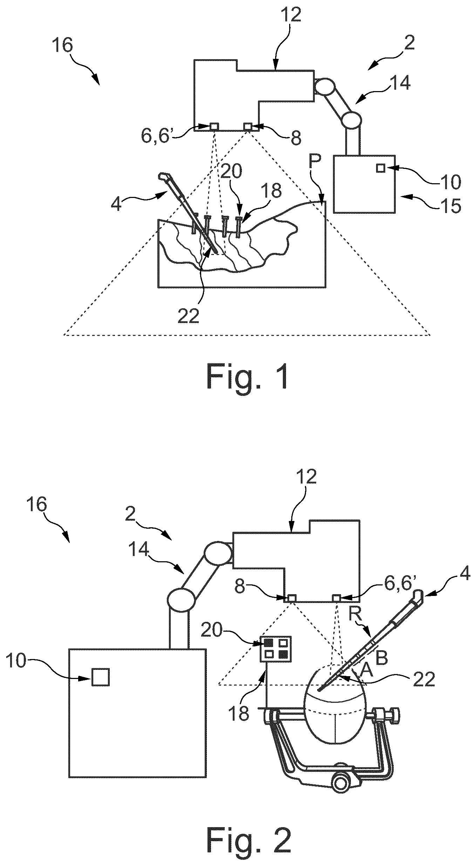

Fig. 1 zeigt ein Navigationssystem 2 zur Nachverfolgung eines medizinischen Instruments 4 relativ zu einem Patienten P mit einer ersten Navigationskamera 6, die eingerichtet ist, eine aktuelle Position und/oder Orientierung des medizinischen Instruments 4 im sichtbaren Spektrum zu erfassen, einer separaten zweiten Navigationskamera 8, die eingerichtet ist, eine aktuelle Position und/oder Orientierung des Patienten P im sichtbaren Spektrum zu erfassen, und mit einer Steuereinheit 10, die eingerichtet ist, aus der von der ersten Navigationskamera 6 erfassten aktuellen Position und/oder Orientierung des medizinischen Instruments 4 und der von der zweiten Navigationskamera 8 erfassten aktuellen Position und/oder Orientierung des Patienten P eine aktuelle Relativposition und/oder -Orientierung des medizinischen Instruments 4 zu dem Patienten P zu bestimmen.

Das Navigationssystem 2 wird hier beispielhaft für einen spinalen Eingriff verwendet.

Die erste Navigationskamera 6 und die zweite Navigationskamera 8 sind gemeinsam und fest zueinander als ein Endeffektor 12 an einem Roboterarm 14 angebracht. Roboterarm 14 ist an einer Basis 15, beispielsweise einem Rollwagen, angebracht.

Die erste Navigationskamera 6 ist ein digitales Operationsmikroskop 6'.

Die zweite Navigationskamera 8 ist eine weitwinklige Kamera. Ein Sichtfeld der zweiten Navigationskamera 8 ist größer als das der ersten Navigationskamera 6. Die Sichtfelder sind gestrichelt angedeutet.

Ein medizinisches System 16 weist das Navigationssystem 2 und ferner einen Patienten-Tracker 18 auf. Der Patienten-Tracker 18 ist dazu eingerichtet ist, an dem Patienten P rigide befestigt zu werden, um dessen Position und/oder Orientierung zu referenzieren. Der Patienten-Tracker 18 weist ein optisches Muster 20 im sichtbaren Spektrum auf. In Fig. 2 ist das optische Muster 20 eines Patienten-Trackers 8 schematisch angedeutet.

Die zweite Navigationskamera 8 ist eingerichtet, das optische Muster 20 des Patienten-Trackers 18 visuell zu erfassen und darüber den Patienten P nachzuverfolgen.

Hier weist das medizinische System 16 mehrere Patienten-Tracker 18 auf, die jeweils an einem Wirbel des Patienten P rigide befestigt sind, um dessen aktuelle Position und/oder Orientierung zu referenzieren bzw. anzugeben.

Das medizinische System 16 weist ferner ein/ das medizinische Instrument 4 auf. Das medizinische Instrument 4 weist ein optisches Muster 22 im sichtbaren Spektrum auf. Die zweite Navigationskamera 6 ist eingerichtet, das optische Muster 22 des medizinischen Instruments 4 visuell zu erfassen und darüber den das medizinische Instrument 4 nachzuverfolgen.

Fig. 2 zeigt das Navigationssystem 2 aus Fig. 1 beispielhaft bei einem neuronalen Eingriff, wobei der Patient P liegt. Der Schädel eines Patienten wird in einer Kopfklemme fixiert. Der Patienten-Tracker 20 ist an dem Schädel rigide befestigt, um dessen Position und/oder Orientierung zu referenzieren.

Das optische Muster 22 des medizinischen Instruments 4 ist im Vergleich zu dem optischen Muster 18 des Patienten-Trackers 20 feiner und kleiner (ausgebildet).

Ferner weist das optische Muster 22 des medizinischen Instruments 4 Redundanzen R auf, um es robuster gegen teilweise Verdeckungen zu machen. Das optische Muster 22 weist Merkmale, hier Striche auf, die sich wiederholen und redundant dieselbe Information (einer Position und/oder Orientierung des medizinischen Instruments 4) bereitstellen. Das optische Muster 22 ist robuster (gegen teilweise Verdeckungen) als das optische Muster 20 des Patienten-Trackers 18, da bei dem Patienten-Tracker 18 die Wahrscheinlichkeit geringer ist, dass er durch z.B. einen Chirurgen oder z.B. Blut des Patienten oder anatomische Strukturen des Patienten verdeckt wird, insbesondere da er außerhalb des Patienten verwendet wird und von dem Operationsgebiet beanstandet sein kann.

Hier stellen die Merkmale A und Merkmale B des optischen Musters 22 dieselbe Information bereit. Wenn die Merkmale A des optischen Musters 22 z.B. durch Blut während der Operation verdeckt sind, kann das medizinischen Instruments 4 weiterhin über die Merkmale B nachverfolgt werden, und umgekehrt genauso. Der Navigationskamera 6 reicht es also aus, eine freie Sicht auf die Merkmale A oder Merkmale B zu haben. Die Redundanzen sind hier schematisch angedeutet und die Figur ist nicht maßstabsgetreu. Es versteht sich, dass die Redundanzen R derart am Instrument 4 angeordnet sind, dass sie gleichzeitig von der Navigationskamera 6 gesehen werden können.

Darüber hinaus weist das optische Muster 22 des Instruments 4 einen niedrigeren Reflexionsgrad als das optische Muster 20 des Patienten-Trackers 18 auf. Das Instrument 4 und das optische Muster 22 des Instruments 4 sind sterilisierbar. Das optische Muster 22 ist eingraviert. Der Patienten-Tracker 18 ist ein Einwegprodukt und kann daher auch nicht sterilisierbar sein. Das optische Muster 20 ist aufgeklebt.

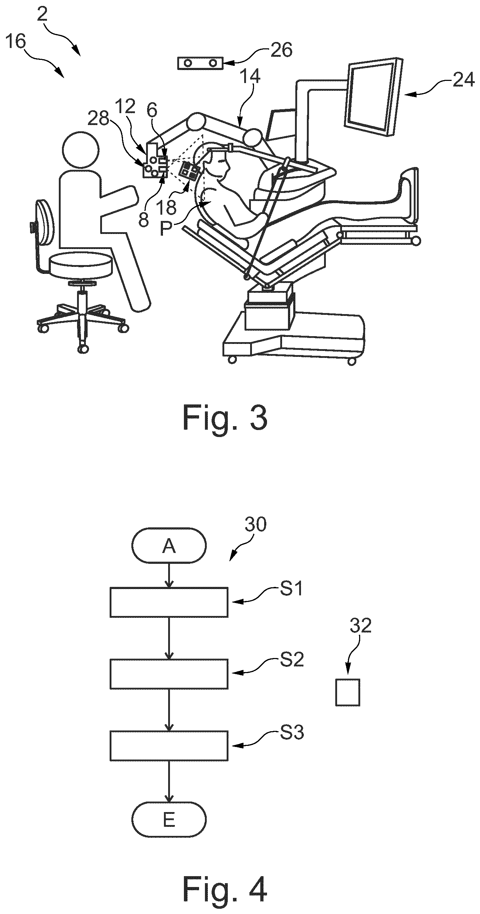

Fig. 3 zeigt ein Navigationssystem 2, ähnlich zu dem aus Fig. 1 und Fig. 2 beispielhaft bei einem Eingriff am Cranium, wobei der Patient P in einer halb-sitzenden (semi sitting) Position ist. Das Navigationssystem 2 weist hier eine Anzeigevorrichtung 24 auf, die eingerichtet ist, eine Aufnahme der ersten Navigationskamera 6 und eine Aufnahme der zweiten Navigationskamera 8 anzuzeigen.

Ferner zeigt Fig. 3 ein medizinisches System 2 ähnlich zu dem aus Fig. 1 und Fig. 2, wobei das medizinische System 2 hier noch eine dritte, separate Navigationskamera 26 für das infrarote (IR) Spektrum und zumindest einen IR-Marker 28, der mit dem Roboterarm 14 verbunden ist, aufweist, wobei die dritte Navigationskamera 26 eingerichtet ist, den IR-Marker 28 im infraroten Spektrum nachzuverfolgen. Hier sind es drei IR-Marker, die an dem Endeffektor 12 angebracht sind. Der Patienten-Tracker 18 ist über eine Kopfklemme rigide an dem Patienten P befestigt bzw. mit ihm rigide verbunden.

Fig. 4 zeigt ein Navigationsverfahren 30. Das Navigationsverfahren 30 ist für eine Nachverfolgung eines medizinischen Instruments 4 relativ zu einem Patienten P und kann beispielsweise von dem Navigationssystem 2 und/oder dem medizinischen System 16 aus Fig. 1 ausgeführt werden. Es hat einen Start A und ein Ende E und weist folgende Schritte S auf:
- (S1): Erfassen einer aktuellen Position und/oder Orientierung des medizinischen Instruments 4 im sichtbaren Spektrum, vorzugsweise über ein optisches Muster 22 des medizinischen Instruments 4, durch eine erste Navigationskamera 6;
- (S2): Erfassen einer aktuellen Position und/oder Orientierung des Patienten P im sichtbaren Spektrum, vorzugsweise über einen Patienten-Tracker 18 mit einem optischen Muster 20, durch eine zweite, separate Navigationskamera 8; und
- (S3): Bestimmen einer aktuellen Relativposition und/oder -Orientierung des medizinischen Instruments 4 zu dem Patienten P aus der von der ersten Navigationskamera 6 erfassten aktuellen Position und/oder Orientierung des medizinischen Instruments 4 und der von der zweiten Navigationskamera 8 erfassten aktuellen Position und/oder Orientierung des Patienten P.

Der Schritt S3 kann z.B. von einer Steuereinheit 12 ausgeführt werden.

Ferner zeigt Fig. 4 ein computerlesbares Speichermedium 32.

### Bezugszeichenliste

- 2: Navigationssystem
- 4: medizinisches Instrument
- P: Patient
- 6: erste Navigationskamera
- 6': digitales Operationsmikroskop
- 8: zweite Navigationskamera
- 10: Steuereinheit
- 12: Endeffektor
- 14: Roboterarm
- 15: Basis
- 16: medizinisches System
- 18: Patienten-Tracker
- 20: optisches Muster (des Patienten-Trackers)
- 22: optisches Muster (des medizinischen Instruments)
- R: Redundanz
- 24: Anzeigevorrichtung
- 26: dritte (IR-) Navigationskamera
- 28: IR-Marker
- 30: Navigationsverfahren
- 32: computerlesbares Speichermedium

## Patentansprüche

1. Navigationssystem (2) zur Nachverfolgung eines medizinischen Instruments (4) relativ zu einem Patienten (P), mit
einer ersten Navigationskamera (6), die eingerichtet ist, eine aktuelle Position und/oder Orientierung des medizinischen Instruments (4) im sichtbaren Spektrum zu erfassen,
einer zweiten Navigationskamera (8), die eingerichtet ist, eine aktuelle Position und/oder Orientierung des Patienten (P) im sichtbaren Spektrum zu erfassen, und
einer Steuereinheit (10), die eingerichtet ist, aus der von der ersten Navigationskamera (6) erfassten aktuellen Position und/oder Orientierung des medizinischen Instruments (4) und der von der zweiten Navigationskamera (8) erfassten aktuellen Position und/oder Orientierung des Patienten (P) eine aktuelle Relativposition und/oder -Orientierung des medizinischen Instruments (4) zu dem Patienten (P) zu bestimmen.

2. Navigationssystem (2) nach Anspruch 1, **dadurch gekennzeichnet, dass** die erste Navigationskamera (6) und die zweite Navigationskamera (8) gemeinsam und fest zueinander, insbesondere als ein Endeffektor (12), an einem Roboterarm (14) angebracht sind.

3. Navigationssystem (2) nach Anspruch 1 oder 2, **dadurch gekennzeichnet, dass** die erste Navigationskamera (6) ein digitales Operationsmikroskop (6') ist.

4. Navigationssystem (2) nach einem der Ansprüche 1 bis 3, **dadurch gekennzeichnet, dass** die zweite Navigationskamera (8) eine weitwinklige Kamera ist, und vorzugsweise ein größeres Sichtfeld als die erste Navigationskamera (6) aufweist.

5. Medizinisches System (16) mit einem Navigationssystem (2) nach einem der Ansprüche 1 bis 4, und ferner mit einem Patienten-Tracker (18), der dazu eingerichtet ist, an dem Patienten (P) rigide befestigt zu werden, um dessen Position und/oder Orientierung zu referenzieren, wobei der Patienten-Tracker (18) ein optisches Muster (20) im sichtbaren Spektrum aufweist und die zweite Navigationskamera (8) eingerichtet ist, das optische Muster (20) des Patienten-Trackers (18) visuell zu erfassen und darüber den Patienten (P) nachzuverfolgen.

6. Medizinisches System (16) nach Anspruch 5, **gekennzeichnet durch** ein medizinisches Instrument (4), wobei das medizinische Instrument (4) ein optisches Muster (22) im sichtbaren Spektrum aufweist und die zweite Navigationskamera eingerichtet ist, das optische Muster (22) des medizinischen Instruments (4) visuell zu erfassen und darüber das medizinische Instrument (4) nachzuverfolgen.

7. Medizinisches System (16) nach Anspruch 6, **dadurch gekennzeichnet, dass** das optische Muster (22) des medizinischen Instruments (4) im Vergleich zu dem optischen Muster (20) des Patienten-Trackers (18) feiner und/oder kleiner ist.

8. Medizinisches System (16) nach einem der Ansprüche 6 oder 7, **dadurch gekennzeichnet, dass** das optische Muster (22) des medizinischen Instruments (4) einen niedrigeren Reflexionsgrad als das optische Muster (20) des Patienten-Trackers (18) aufweist.

9. Medizinisches System (16) nach einem der Ansprüche 6 bis 8, **dadurch gekennzeichnet, dass** das optische Muster (22) des medizinischen Instruments (4) für eine Wiederverwendbarkeit und Sterilisierbarkeit eingraviert und/oder beschichtet ist, wobei vorzugsweise der Patienten-Tracker (18) ein Einwegprodukt ist und/oder vorzugsweise das optische Muster (20) des Patienten-Trackers (18) nicht sterilisierbar ist.

10. Medizinisches System (16) nach einem der Ansprüche 6 bis 9, **dadurch gekennzeichnet, dass** das optische Muster (22) des medizinischen Instruments (4), insbesondere im Vergleich zu dem optischen Muster (20) des Patienten-Trackers (18), robust gegen eine teilweise Verdeckung ist, vorzugsweise durch, insbesondere mehr, Redundanzen (R).

11. Medizinisches System (16) nach einem der Ansprüche 5 bis 10, **gekennzeichnet durch** eine dritte Navigationskamera (26) für das infrarote (IR) Spektrum und zumindest einen IR-Marker (28), der mit dem Roboterarm (14) verbunden ist, wobei die dritte Navigationskamera (26) eingerichtet ist, den IR-Marker (14) im infraroten Spektrum nachzuverfolgen.

12. Navigationsverfahren (30) zur Nachverfolgung eines medizinischen Instruments (4) relativ zu einem Patienten (P), vorzugsweise für ein Navigationssystem (2) nach einem der Ansprüche 1 bis 4 oder ein medizinisches System (16) nach einem der Ansprüche 5 bis 11, mit den Schritten (S):
- (S1): Erfassen einer aktuellen Position und/oder Orientierung des medizinischen Instruments (4) im sichtbaren Spektrum, vorzugsweise über ein optisches Muster (22) des medizinischen Instruments (4), durch eine erste Navigationskamera (6);
- (S2): Erfassen einer aktuellen Position und/oder Orientierung des Patienten (P) im sichtbaren Spektrum, vorzugsweise über einen Patienten-Tracker (18) mit einem optischen Muster (20), durch eine zweite, separate Navigationskamera (8); und
- (S3): Bestimmen einer aktuellen Relativposition und/oder -Orientierung des medizinischen Instruments (4) zu dem Patienten (P) aus der von der ersten Navigationskamera (6) erfassten aktuellen Position und/oder Orientierung des medizinischen Instruments (4) und der von der zweiten Navigationskamera (8) erfassten aktuellen Position und/oder Orientierung des Patienten (P).

13. Verwendung einer ersten Navigationskamera (6), insbesondere eines Navigationssystems (2) nach einem der Ansprüche 1 bis 4 oder eines medizinisches Systems (16) nach einem der Ansprüche 5 bis 11, zum Erfassen einer aktuellen Position und/oder Orientierung eines medizinischen Instruments (4) im sichtbaren Spektrum und einer separaten zweiten Navigationskamera (8), insbesondere eines Navigationssystems (2) nach einem der Ansprüche 1 bis 4 oder eines medizinisches Systems (16) nach einem der Ansprüche 5 bis 11, zum Erfassen einer aktuellen Position und/oder Orientierung eines Patienten (P) im sichtbaren Spektrum, um das medizinische Instrument (4) relativ zu dem Patienten (P) nachzuverfolgen.
